# EUROPEAN PATENT SPECIFICATION

(11) **EP 0 550 640 B1**
(45) Date of publication and mention of the grant of the patent: **26.03.1997**
(21) Application number: 91918129.7
(22) Date of filing: 25.09.1991
(51) Int. Cl.: B01J 27/24, B01J 27/186, B01J 27/187, C07C 2/58, B01J 27/199, B01J 27/135, B01J 21/02, B01J 27/08, C07C 2/60, B01J 27/12, B01J 27/125

(54) **LEWIS ACID PROMOTED TRANSITION ALUMINA CATALYSTS AND ISOPARAFFIN ALKYLATION PROCESSES USING THOSE CATALYSTS**
Durch Lewissäure aktivierte Übergangs- Aluminiumoxydkatalysatoren und Verfahren zur Alkylierung von Isoparaffinen unter Verwendung dieser Katalysatoren
Catalyseurs d'alumine de transition activés par les acides Lewis et procédés d'alkylation des paraffines utilisant ces catalyseurs.

(30) Priority: 26.09.1990 US 588448; 07.05.1991 US 697318; 07.05.1991 US 697320
(43) Date of publication of application: 14.07.1993
(73) Proprietor: CATALYTICA, INC., Mountain View, CA 94043-5272 (US)
(72) Inventor: COOPER, Michael, D., San Jose, CA 95126 (US); KING, David, L., Mountain View, CA 94040 (US); SANDERSON, William, A., Portola Valley, CA 94028 (US)
(74) Representative: Goldin, Douglas Michael
(86) International application number: PCT/US91/06999
(87) International publication number: WO 92/04977

(56) References cited:
- WO-A-90/00534
- FR-A- 1 346 135
- FR-A- 2 504 121
- SU-A- 614 079
- US-A- 2 405 565
- US-A- 2 406 869
- US-A- 2 804 491
- US-A- 2 939 890
- US-A- 3 054 835
- US-A- 3 054 836
- US-A- 3 068 301
- US-A- 3 114 785
- US-A- 3 217 062
- US-A- 3 217 063
- US-A- 3 220 958
- US-A- 3 433 747
- US-A- 4 407 731
- US-A- 4 918 255

## Description

### FIELD OF THE INVENTION

This invention is to a catalyst system comprising certain transition aluminas promoted with a Lewis acid (preferably BF₃), and b.) a catalytic process for the alkylation of isoparaffin with olefins. A catalyst component is produced by contacting the transition alumina with the Lewis acid at relatively low temperatures. The catalyst system comprises that component and an additional amount of free Lewis acid. The process entails olefin/isoparaffin alkylation using the catalyst component and its allied catalyst system.

### BACKGROUND OF THE INVENTION

The preparation of high octane blending components for motor fuels using strong acid alkylation processes (notably where the acid is hydrofluoric acid or sulfuric acid) is well-known. Alkylation is the reaction in which an alkyl group is added to an organic molecule, typically an aromatic or olefinic molecule. For production of gasoline blending stocks, the reaction is between an isoparaffin and an olefin. Alkylation processes have been in wide use since World War II when high octane gasolines were needed to satisfy demands from high compression ratio or supercharged aircraft engines. The early alkylation units were built in conjunction with fluid catalytic cracking units to take advantage of the light end by-products of the cracking units: isoparaffins and olefins. Fluidized catalytic cracking units still constitute the major source of feedstocks for gasoline alkylation units. In spite of the mature state of strong acid alkylation technology, existing problems with the hydrofluoric and sulfuric acid technologies continue to be severe: disposal of the used acid, unintentional emission of the acids during use or storage, substantial corrosivity of the acid catalyst systems, and other environmental concerns.

Although a practical alkylation process using solid acid catalysts having little or no corrosive components has long been a goal, commercially viable processes do not exist.

The open literature shows several systems used to alkylate various hydrocarbon feedstocks.

The American Oil Company obtained a series of patents in the mid-1950's on alkylation processes involving C₂-C₁₂ (preferably C₂ or C₃) olefins and C₄-C₈ isoparaffins. The catalysts used were BF₃-treated solids and the catalyst system (as used in the alkylation process) also contained free BF₃. A summary of those patents is found in the following list:

| Patent No. | Inventor | BF₃-Treated Catalyst* (with free BF₃) |
|---|---|---|
| 2,804,491 | May et al. | SiO₂ stabilized Al₂O₃ (10%-60% by weight BF₃) |
| 2,824,146 | Kelly et al. | metal pyrophosphate hydrate |
| 2,824,150 | Knight et al. | metal sulfate hydrate |
| 2,824,151 | Kelly et al. | metal stannate hydrate |
| 2,824,152 | Knight et al. | metal silicate hydrate |
| 2,824,153 | Kelly et al. | metal orthophosphate hydrate |
| 2,824,154 | Knight et al. | metal tripolyphosphate hydrate |
| 2,824,155 | Knight et al. | metal pyroarsenate hydrate |
| 2,824,156 | Kelly et al. | Co or Mg arsenate hydrate |
| 2,824,157 | Knight et al. | Co, Al, or Ni borate hydrate |
| 2,824,158 | Kelly et al. | metal pyroantimonate hydrate salt |
| 2,824,159 | Kelly et al. | Co or Fe molybdate hydrate |
| 2,824,160 | Knight et al. | Al, Co, or Ni tungstate hydrate |
| 2,824,161 | Knight et al. | borotungstic acid hydrate or Ni or Cd borotungstate hydrate |
| 2,824,162 | Knight et al. | phosphomolybdic acid hydrate |
| 2,945,907 | Knight et al. | solid gel alumina (5%-100% by weight of Zn or Cu fluoborate, preferably anhydrous) |

| | | |
|---|---|---|
| *may be supported on Al₂O₃ | | |

None of these discloses a process for alkylating olefins and isoparaffins using neat alumina treated with BF₃.

Related catalysts have been used to oligomerize olefins. U.S. Patent No. 2,748,090 to Watkins suggests the use of a catalyst made up of a Group VIII metal (preferably nickel), a phosphoric acid (preferably containing phosphorus pentoxide), all placed on an alumina adsorbent, and pretreated with BF₃. Alkylation of aromatics is suggested.

U.S. Patent No. 2,976,338 to Thomas suggests a polymerization catalyst comprising a complex of BF₃ or H₃PO₄ optionally on an adsorbent (such as activated carbon) or a molecular sieve optionally containing potassium acid fluoride.

Certain references suggest the use of alumina-containing catalysts for alkylation of aromatic compounds. U.S. Patent No. 3,068,301 to Hervert et al. suggests a catalyst for alkylating aromatics using "olefin-acting compounds". The catalyst is a solid, silica-stabilized alumina containing up to 10% SiO₂, all of which has been modified with up to 100% by weight of BF₃. None of these prior references suggest either the process nor the material used in the processes as is disclosed here.

Other BF₃-treated aluminas are known. For instance, U.S. Patent No. 3,114,785 to Hervert et al. suggests the use of a BF₃-modified, substantially anhydrous alumina to shift the double bond of 1-butene to produce 2-butene. The preferred alumina is substantially anhydrous gamma-alumina, eta-alumina, or theta-alumina. The various aluminas will adsorb or complex with up to about 19% by weight fluorine depending upon the type of alumina and the temperature of treatment. The aluminas are treated with BF₃ at elevated temperatures. Hervert et al. does not suggest using these catalysts in alkylation reactions.

In U.S. Patent No. 4,407,731 to Imai, a high surface area metal oxide such as alumina (particularly gamma-alumina, eta-alumina, theta-alumina, silica, or a silica-alumina) is used as a base or support for BF₃. The BF₃ treated metal oxide is used for generic oligomerization and alkylation reactions. The metal oxide is treated in a complicated fashion prior to being treated with BF₃. The first step entails treating the metal oxide with an acid solution and with a basic aqueous solution. The support is washed with an aqueous decomposable salt such as ammonium nitrate. The support is washed using deionized H₂O until the wash water shows no alkali or alkaline earth metal cations in the filtrate. The support is dried and calcined. The disclosure suggests generically that BF₃ is then introduced to the treated metal oxide support. The examples show introduction of the BF₃ at elevated temperatures, e.g, 300° C or 350° C.

Similarly, U.S. Patent No. 4,427,791 to Miale et al. suggests the enhancement of the acid catalytic activity of inorganic oxide materials (such as alumina or gallia) by contacting the material with ammonium fluoride or boron fluoride, contacting the treated inorganic oxide with an aqueous ammonium hydroxide or salt solution, and calcining the resulting material. The inorganic oxides treated in this way are said to exhibit enhanced Brönsted acidity and, therefore, are said to have improved acid activity towards the catalysis of numerous reactions (such as alkylation and isomerization of various hydrocarbon compounds). A specific suggested use for the treated inorganic oxide is as a matrix or support for various zeolite materials ultimately used in acid catalyzed organic compound conversion processes.

U.S. Patent No. 4,751,341 to Rodewald shows a process for treating a ZSM-5 type zeolite with BF₃ to reduce its pore size, enhance its shape selectivity, and increase its activity towards the reaction of oligomerizing olefins. The patent also suggests using these materials for alkylation of aromatic compounds.

Certain Soviet publications suggest the use of Al₂O₃ catalysts for alkylation processes. Benzene alkylation using those catalysts (with 3 ppm to 5 ppm water and periodic additions of BF₃) is shown in Yagubov, Kh. M. et al., Azerb. Khim. Zh., 1984, (5) p. 58. Similarly, Kozorezov, Yu and Levitskii, E.A., Zh. Print. Khim. (Leningrad), 1984, 57 (12), p. 2681, show the use of alumina which has been heated at relatively high temperatures and modified with BF₃ at 100° C. There are no indications that BF₃ is maintained in excess. Isobutane alkylation using Al₂O₃/BF₃ catalysts is suggested in Neftekhimiya, 1977, 17 (3), p. 396; 1979, 19 (3), p. 385. The olefin is ethylene. There is no indication that BF₃ is maintained in excess during the reaction. The crystalline form of the alumina is not described.

U.S. Patent No. 4,918,255 to Chou et al. suggests a process for the alkylation of isoparaffins and olefins using a composite described as "comprising a Lewis acid and a large pore zeolite and/or a non-zeolite inorganic oxide". The process disclosed requires isomerization of the olefin feed to reduce substantially the content of α-olefin and further suggests that water addition to the alkylation process improves the operation of the process. The best Research Octane Number (RON) product made using the inorganic oxides (in particular SiO₂) is shown in Table 6 to be 94.0.

Similarly, PCT published applications WO 90/00533 and 90/00534 (which are based in part on the U.S. patent to Chou *et al*. noted above) suggest the same process as does Chou *et al*. WO 90/00534 is specific to a process using boron trifluoride-treated inorganic oxides including "alumina, silica, boria, oxides of phosphorus, titanium oxide, zirconium oxide, chromia, zinc oxide, mangesia, calcium oxide, silica-alumina-zirconia, chromia-alumina, alumina-boria, silica-zirconia, and the various naturally occurring inorganic oxides of various states of purity such as bauxite, clay and diatomaceous earth". Of special note is the statement that the "preferred inorganic oxides are amorphous silicon dioxide and aluminum oxide". The examples show the use of amorphous silica (and BF₃) to produce alkylates having a RON of no greater than 94.

SU-A-614079 describes the alkylation of isobutane in the liquid phase using a solid Al₂O₃ catalyst containing absorbed BF₃. The catalyst is prepared by calcining Al₂O₃ at 500 to 600°C to 1 to 1.5 by weight percent moisture content adding BF₃ at 20 to 30°C and pumping off non-absorbed BF₃.

GB-A-1025896 describes a process for polymerising an olefinic hydrocarbon which comprises introducing an olefinic hydrocarbon reactant stream into a zone having a catalyst bed comprising a boron trifluoride modified substantially anhydrous alumina and separately introducing a boron trifluoride stream. A greater yield of the polymer product is obtained if the olefinic hydrocarbon is not allowed to come into contact with the boron trifluoride before the boron trifluoride contacts the alumina.

US-A-2939890 describes a process for alkylating aromatic hydrocarbons, particularly benzene hydrocarbons. The aromatic hydrocarbon is passed to an alkylation zone containing boron trifluoride modified substantially anhydrous γ- or θ-alumina and not more than 0.8 g of boron trifluoride per gram mole of olefin. The boron trifluoride-modified alumina is prepared by contacting the alumina with from about 2 % to about 100 % by weight of boron trifluoride based on the alumina. The contact is carried out at temperatures up to about 300°C.

US-A-2804491 describes the alkylation of isobutane with ethylene in the presence of a catalyst pair which comprises boron trifluoride and a solid silica stabilised gel alumina containing between about 10 and 60 weight percent of BF₃ based on alumina.

None of these disclosures shows crystalline transition aluminas which were promoted with Lewis acids at lower temperatures nor any effect upon the NMR spectrum because of such a treatment. Nor do these disclosures show their use in isoparaffin/olefin alkylation. These disclosures further do not show any benefit to the alkylation of isoparaffins and olefins using these specifically treated aluminas.

### BRIEF DESCRIPTION ON THE DRAWINGS

Figures 1A-1D are nuclear magnetic resonance (NMR) plots of certain transition aluminas treated with BF₃ at a range of temperatures.

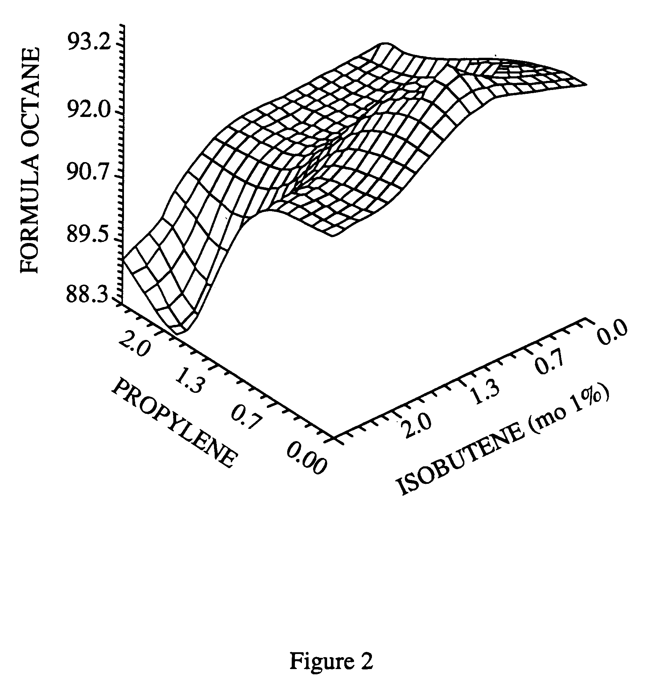

Figure 2 is a three-dimensional graph showing octane sensitivity for the inventive process as a function of olefin feed content.

### SUMMARY OF THE INVENTION

This invention is variously a catalyst system which comprises a catalyst component comprising one or more transitional aluminas which are treated with one or more Lewis acids (preferably BF₃) at a fairly low temperature desirably so low that the component exhibits specific NMR spectra, a catalyst system comprising that catalyst component with excess Lewis acid, and an olefin/isoparaffin alkylation process step using that catalyst system.

Use of the catalyst system, i.e. the catalyst component in conjunction with excess Lewis acid, produces high octane alkylate from isobutane and butylene at a variety of reaction temperatures between -30°C and 40°C. The catalyst's high activity can result in low operating costs because of its ability to operate at high space velocities.

### DESCRIPTION OF THE INVENTION

This invention is:
(A) a catalyst system comprising the catalyst component in combination with at least a minor amount of free Lewis acid, and
(B) an alkylation process for producing branched paraffinic products from olefins and isoparaffins using that catalyst system.

### The Catalyst Component

The catalyst component used in the catalyst system of this invention comprises or consists essentially of a major amount of transition aluminas (preferably eta- or gamma-alumina) which has been treated with a Lewis acid, preferably BF₃. The catalyst component is acidic in nature and contains substantially no metals (except, of course, aluminum and the semi-metal boron), in catalytic amounts capable of hydrogenating the hydrocarbons present in the feeds except those metals may be present in trace amounts in the Lewis acid or the alumina.

### Alumina

Aluminum oxide (alumina) occurs abundantly in nature, usually in the form of a hydroxide in the mineral bauxite, along with other oxidic impurities such as TiO₂, Fe₂O₃, and SiO₂. The Bayer process is used to produce a reasonably pure Al₂O₃ having a minor amount of Na₂O. The Bayer process may be used to produce a variety of alumina hydroxides:

| Material | Common Name | %H₂O | H₂O/Al₂O₃ | CAS Index No. |
|---|---|---|---|---|
| α-trihydrate | hydrargillite/gibbsite | 35 | 3.0 | 14762-493 |
| β-trihydrate | bayerite | 35 | 3.0 | 20257-20-9 or 12252-72-1 |
| β-trihydrate | nordstrandite | 35 | 3.0 | 13840-05-6 |
| α-monohydrate | boehmite | 15 | 1.0 | 1318-23-6 |
| hydrate | psuedoboehmite | 26 | 2.0 | ― |

The aluminum hydroxides may then be treated by heating to produce various activated or transition aluminas. For instance, the aluminum hydroxide known as boehmite may be heated to form a sequence of transition phase aluminas: gamma, delta, theta, and finally, alpha (see Wefers et al., "Oxides and Hydroxides of Alumina", Technical Paper No. 19, Aluminum Company of America, Pittsburgh, PA, 1972, pp.1-51).

Transition aluminas (and their crystalline forms) include:
- gamma: tetragonal
- delta: orthorhombic/tetragonal
- eta: cubic
- theta: monoclinic
- chi: cubic/hexagonal
- kappa: hexagonal
- lambda: orthorhombic
Activated aluminas and aluminum hydroxides are used in various chemical processes as catalyst and adsorbents.

The aluminas suitable for use in this process include the noted transition aluminas: gamma, delta, eta, theta, chi, kappa, or lambda. Especially preferred are gamma- and eta-aluminas. Mixtures of the two are also desireable.

Since it is difficult to produce a substantially pure single phase transition alumina, mixtures of various aluminas are tolerable so long as a major amount of the specified alumina is present in the catalyst. For instance, in the production of eta-alumina, gamma-alumina is often concurrently present in the resulting product. Indeed, x-ray diffraction analysis can only difficulty detect the difference between the two phases. Aluminum hydroxides (boehmite, gibbsite, etc.) may be present in the predominately transition phase product in more than trivial amounts so long as they do not substantially effect the desired alkylation reaction.

The alumina may be produced in any appropriate form such as pellet, granules, bead, sphere, powder, or other shape to facilitate its use in fixed bed, moving bed, slurry, or fluidized bed reactors.

### Lewis Acids

The catalyst component used in this invention contains one or more Lewis acids in conjunction with the alumina noted above. A Lewis acid is a molecule which can form another molecule or an ion by forming a complex in which it accepts two electrons from a second molecule or ion. Typical strong Lewis acids include boron halides such as BF₃, BCl₃, BBr₃, and BI₃; antimony pentafluoride (SbF₅); aluminum halides (AlCl₃ and AlBr₃); titanium halides such as TiBr₄, TiCl₄, and TiCl₃; zirconium tetrachloride (ZrCl₄); phosphorus pentafluoride (PF₅); iron halides such as FeCl₃ and FeBr₃; and the like. Weaker Lewis acids such as tin, indium, bismuth, zinc, or mercury halides are also acceptable. Preferred Lewis acids are boron containing materials (BF₃, BCl₃, BBr₃, and BI₃), SbF₅, and AlCl₃; most preferred is BF₃.

The Lewis acid typically forms complexes or surface compounds with the alumina substrate. For instance, BF₃ forms aluminum fluoroborate sites with the hydroxyl groups found at the alumina surface and additionally is physi-sorbed at the alumina surface.

The total amount of Lewis acid in the alumina surface is between 0.5% and 40% by weight of the catalyst depending in large measure on two factors: the Lewis acid chosen and the susceptibility of the alumina surface to accepting the Lewis acid by chemisorption or by physisorption. In the case of BF₃, we believe that 5-20% of the weight of the alumina catalyst component is attributable to BF₃ products (e.g., the production of aluminum fluoroborate or similar compounds) and the remainder is physi-sorbed BF₃.

To maintain the presence of sufficient Lewis acid on the catalyst composition, we have found it desirable to maintain at least a minor amount of the Lewis acid in the proximity of the alumina surface, preferably in the reaction fluid. This amount is an amount at least sufficient to maintain the concentration of the Lewis acid specified above on the alumina. At the WHSV ranges specified above with regard to the alkylation reaction, we have found that generally an amount of at least 0.5% of Lewis acid (based on the hydrocarbon) is sufficient to maintain the Lewis acid level on the alumina. On an alumina basis, the ratio of free Lewis acid (that is, Lewis acid in the proximity of the alumina but not associated with the alumina by chemisorption or physisorption) to alumina is in the range of 0.05 to 25 g Lewis acid/g Al₂O₃. For BF₃ the preferred range is 0.15 to 20 g BF₃/g Al₂O₃, is more preferably in the range of 0.20 to 15 g BF₃/g Al₂O₃, and is most preferably in the range of 0.10 to 15 g BF₃/g Al₂O₃.

### Catalyst Component Preparation

The catalyst component may be prepared in situ in, e.g., an alkylation reactor by passing the Lewis acid in gaseous form through the vessel containing the transition alumina. Alternatively, the alumina may be contacted with the Lewis acid and later introduced into the reactor.

In any case, the alumina may be substantially dry or anhydrous prior to contact with the Lewis acid and maintained in a state of dryness, i.e., maintained at a very low free H₂O content. The alumina phase chosen in conjunction with proper treatment of the alumina to maintain the presence of hydroxyl groups (usually by maintaining the alumina at temperatures below 450 °C during pretreatment) allows the presence of about 4-10 hydroxyl groups per 100 Å² of alumina surface area. Preferred is 6-10 hydroxyl groups per 100 Å² of alumina surface area. The alumina is preferably completely hydroxylated since that hydroxylation, in turn, permits the formation of the maximum amount of the Al-OH-Lewis acid complex, believed to be one element of the active alkylation catalyst at the alumina surface. The alumina may be partially or substantially dehydroxylated but the catalyst is not as efficacious.

Additionally, free water (in distinction to the water which may be identified as hydroxyl groups on the alumina surface) may be present in limited amounts in the alumina. The free water content in the alumina may range between 0.0 and 10% by weight but preferably is between about 0.0 and about 5.0 %. If the Lewis acid chosen is BF₃, the free water content of the alumina may be between 0.0 and 4.0 % (by weight), preferably between 1.0 and 3.5 %, and most preferably between 2.5 and 3.5 %. Higher amounts of water appear both to degrade the catalyst and to impair the effectiveness of the catalyst in the practice of the alkylation reaction. Higher amounts of water also tend to form compounds, such as BF₃ hydrates, which are corrosive and therefore undesirable.

Contact temperatures between -25° C and less than about 150° C are acceptable; a temperature between -25° C and 100° C is desirable; a temperature between -30° C and 30° C is preferred. The partial pressure of gaseous Lewis acid added to the alumina is not particularly important so long as a sufficient amount of Lewis acid is added to the alumina. We have found that treatment of the alumina with BF₃ at the noted temperatures will result in an alumina-BF₃ complex containing BF₃ sufficient to carry out the alkylation. The alumina contains between 0.5% and 30% by weight of BF₃. We have observed that solid state boron-nuclear magnetic resonance (¹¹B-NMR) analysis of the catalyst component provides evidence (a pronounced peak at about -21.27 ppm relative to boric acid) of tetragonal boron in the catalyst composite produced at the lower temperatures. Aluminas treated at temperatures of 150°C and higher do not show these spectra but instead show evidence of trigonal symmetry about the boron. Acceptably active catalysts are those in which the relative amounts of trigonal boron:tetragonal boron (as calculated by the integration of the respective ¹¹B-NMR spectra) are in the range of 0 to 0.5. The lower the value of the ratio, the more effective the catalyst has been found to be in alkylation reactions. More preferred is the range of 0.0 to 0.25; most preferred is 0.0 to 0.1.

Additionally, we have observed that when the alumina substrates are heated at temperatures above about 400° C before they are contacted with the Lewis acid BF₃, that certain areas of the infrared spectra are modified. The Fourier Transform Infrared (FTIR) absorbances at about 1557 and 1510 cm⁻¹ which we observe when the alumina is treated at temperatures below that level disappear above this temperature. Although we believe that the disappearance of those IR spectral absorbances is somehow related to the decomposition of the key surface intermediates involved in the catalytic function, we do not wish to be bound to this theory.

Obviously, the alumina may be incorporated into a binder prior to its treatment with Lewis acid. The binders may be clays (such as montmorillonite and kaolin) or silica based materials (such as gels or other gelatinous precipitates). Other binder materials include carbon and metal oxides such as alumina, silica, titania, zirconia, and mixtures of those metal oxides. The composition of the binders is not particularly critical but care must be taken that they not substantially interfere with the operation of the alkylation reaction.

The preferred method for incorporating the catalytic alumina into the binder is by mixing an aluminum hydroxide precursor (such as boehmite) with the binder precursor, forming the desired shape, and calcining at a temperature which both converts the aluminum hydroxide precursor into the appropriate transition phase and causes the binder precursor to bind the alumina particles. The absolute upper temperature limit for this calcination is about 1150° C. Temperatures below about 1000° C may be appropriate.

### Alkylation Process

The inventive catalyst system is especially suitable for use in alkylation processes involving the contact of an isoparaffin with an olefin.

Specifically, the catalyst system (the catalyst component in combination with a free Lewis acid) is active in alkylation reactions at low temperatures (as low as -30° C) as well as at higher temperatures (nearing 50° C). Lower temperatures (-5° C to 15° C) are preferred because of the enhanced octane of the alkylate produced and are particularly preferred if the feedstream contains more than about 1% isobutylene. Higher temperatures also tend to produce larger amounts of polymeric materials.

The pressure used in this process may be between atmospheric pressure and about 750 psig (5171 kPa). Higher pressures within the range allow recovery of excess reactants by flashing after the product stream leaves the alkylation reactor. The amount of catalyst used in this process depends upon a wide variety of disparate variables. Nevertheless, the Weight Hourly Space Velocity ("WHSV" = weight of olefin feed/hour + weight of catalyst) may effectively be between 0.1 and 120, especially between 0.5 and 30. The overall molar ratio of isoparaffin to olefin may be between about 1.0 and 50.0. Preferred ranges include 2.0 and 25.0; the more preferred include 3.0 and 15.0.

The feedstreams introduced to the catalyst are desirably chiefly isoparaffins having from four to ten carbon atoms and, most preferably, four to six carbon atoms. Isobutane is most preferred because of its ability to make high octane alkylate. The olefins desirably contain from three to twelve and preferably from three to five carbon atoms, i.e., propylene, cis- and trans-butene-2, butene-1, and amylene(s). Preferably, the olefin stream contains little (if any) isobutylene. Similarly, for the inventive catalysts the process works better in producing high octane alkylate if the feedstream contains little or no butadiene (preferably less than 0.2% to 0.3% molar of the total olefins) and a minimal amount of isobutylene, e.g., less than about 2.5% molar based on the olefins. Although the catalyst alkylates butene-1, it is preferred to operate with a minimum of butene-1, e.g., less than about 10% by mol, since it lowers the octane values of the resulting alkylate. Of course, if it is desired to operate a process with high throughput rather than with highest octane, a higher level of butene-1 is tolerable. An excellent source of a feedstock containing a low level of isobutylene is the raffinate from a process which produces methyl-t-butylether (MTBE).

The water content of the feedstocks may vary within wide limits, but preferably is at a low level. The water content should be less than about 200 ppmw and most preferably less than about 50 ppmw. Higher levels of water content tend to lower the octane value of the resulting alkylate and form corrosive hydrates or reaction products with the Lewis acids.

The feedstocks should contain a minimum of oxygenates such as ethers and alcohols. Oxygenates appear to lessen substantially the effectiveness of the catalyst system.

The products of this alkylation process typically contain a complex mixture of highly branched alkanes. For instance, when using isobutane as the alkane and n-butylene as the olefin, a mixture of 2,2,3-; 2,2,4-; 2,3,3-; and 2,3,4-trimethylpentane (TMP) will result often with minor amounts of other isomeric or polymeric products. The 2,3,4-TMP isomer is the lowest octane isomer of the noted set. The 2,2,3- and 2,2,4-TMP isomers are higher octane components. Calculated average octane values (RON plus Motor Octane Number [MON]/2) of the various C₈ isomers are:

| Isomer | Octane (R + M)/2 |
|---|---|
| 2,2,3- | 104.8 |
| 2,2,4- | 100.0 |
| 2,3,3- | 102.8 |
| 2,3,4- | 99.3 |

The process may be carried out in the liquid, vapor, or mixed liquid and vapor phase. Liquid phase operation is preferred.

The invention has been disclosed by direct description. Below may be found a number of examples showing various aspects of the invention. The examples are only examples of the invention and are not to be used to limit the scope of the invention in any way.

### EXAMPLES

### Example 1: Catalyst Testing

This example shows the preparation of a number of alumina-based catalysts in situ and their subsequent use in an alkylation reaction using model feeds. It is used to evaluate catalyst activity and selectivity.

The alumina samples were dried at 150° C overnight and charged to a semi-batch reactor having an internal volume of about 500 cc. The reactor temperature was controllable over the range of -5° C to 40° C. For initial catalyst treatment, the reactor containing the catalyst was purged with an inert gas and cooled to about 0° C. About 275 cc of isobutane was added to the reactor. After a brief degassing, BF₃ was added batchwise. After BF₃ is added, the pressure typically drops as the alumina adsorbs or reacts with the BF₃. Additional infusions of BF₃ are made until the pressure in the reactor no longer drops. The BF₃ saturation equilibrium pressure was about 40 psig. The liquid phase concentration of BF₃ was about 1.5%. At that point the alumina had adsorbed or reacted with all of the BF₃ possible at that temperature and the catalyst was in its most active form.

A 4/1 molar mixture of isobutane and trans-2-butene was added to the reactor at a WHSV of 3.5 until the paraffin to olefin ratio reached 25.

The product alkylate was then removed from the reactor vessel and analyzed using gas-liquid chromatography.

The results of those runs are shown in Table I.

**Table 1**

| Alumina Type | Surface Area | %C₈ in Alkylate Product |
|---|---|---|
| gamma | 180 m²/gm | 95.4 |
| gamma | 116 m²/gm | 82.07 |
| delta | 118 m²/gm | 94.3 |
| pseudoboehmite | 352 m²/gm | 74.2 |
| bayerite | 40 m²/gm | 69.1 |
| pseudoboehmite | 250 m²/gm | 59.6 |
| boehmite | 150 m²/gm | 59.8 |

It is clear from these preliminary screening data that the transition (gamma and delta) aluminas produce significantly higher percentages of C₈ in the product alkylate than do the other aluminum hydroxide catalysts. The result did not appear to correlate to the specific surface area of the catalyst.

### Example 2: Catalyst Screening

This example compares the performance of eta-alumina (a preferred form of the inventive catalyst) with representative samples of other acidic oxides each combined with BF₃ for the reaction of isobutane with butenes to produce alkylate.

The eta-alumina sample was prepared by a controlled thermal treatment of bayerite (Versal B from LaRoche Chemical) for 15 hours at 250° C and 24 hours at 500° C under a N₂ atmosphere.

The comparative oxidic materials were: silica-alumina, synthetic mordenite zeolite, and fumed silica. The silica-alumina (obtained from Davison Chemical) contained 86.5% SiO₂ and had a surface area of 392 m²/gm. It was used without further treatment.

The mordenite was a hydrogen form zeolite and was obtained from Toyo Soda. It was prepared from Na-mordenite and subjected to ion exchange, steam treatment, and calcination to achieve a Si/Al ratio of 28.

Each of the samples was dried at 150° C overnight and introduced into the semi-batch reactor described in Example 1. The samples were purged with a dry inert gas and cooled to 0° C. Isobutane was added to the reactor to an initial volume of 100 cc. BF₃ was added with stirring until an equilibrium pressure of 30 psig was obtained.

A mixture of isobutane/t-2-butene was fed to the reactor. At the completion of the reaction, alkylate was removed and analyzed by gas-liquid chromatography. The RON were calculated from the gas-liquid chromatography data using the well-known correlations in Hutson and Logan, "Estimate Alkyl Yield and Quality", Hydrocarbon Processing, September, 1975, pp. 107-108. The summary of the experiments and results is shown in the table below:

**Table 2**

| | Eta-Al₂O₃ | Silica-Alumina | Dealuminated Mordenite | Silica |
|---|---|---|---|---|
| Catalyst charge (g) | 3.5 | 3.7 | 2.4 | 1.8 |
| Temperature (°C) | 0 | 0 | 0 | 0 |
| i-C₄ charge ml (initial) | 180 | 180 | 180 | 375 |
| i-C₄/C₄⁼feed ratio (molar) | 5.2 | 5.9 | 5.9 | 9.5 |
| Space velocity (WHSV) | 2.6 | 2.0 | 3.3 | 2.8 |
| Run time (minutes) | 36 | 34 | 28 | 58 |
| i-C₄/C₄₌(final) | 23.5 | 30.3 | 34.0 | 57 |
| Butene conversion (%) | 100 | 100 | 100 | 100 |

| Product analysis (weight %): | | | | |
|---|---|---|---|---|
| C₅-C₇ | 3.1 | 5.2 | 11.3 | 13.0 |
| C₈ saturates | 95.7 | 75.8 | 71.7 | 70.9 |
| C₉⁺ | 1.2 | 19.0 | 17.0 | 16.1 |
| TMP/C₈ total (%) | 93.0 | 91.2 | 91.3 | 91.6 |
| Yield (w/w) | 2.08 | 1.55 | 0.99 | 1.43 |
| RON | 99.3 | 94.6 | 93.0 | 93.0* |
| Octane (R+M/2) | 97.9 | 93.1 | 92.0 | 92.1* |

| | | | | |
|---|---|---|---|---|
| *estimated | | | | |

Clearly, for the eta-alumina catalyst, the yield of C₈'s was significantly higher; the overall yield and RON were much better.

### Example 3

This example shows that the addition of either water or methanol produces no appreciable improvement on the alkylation of butene-2 with isobutane using the inventive alumina catalyst. Indeed, water and methanol appear to be detrimental.

Three separate semi-batch reactors were dried and flushed with nitrogen. A sample of 2.5 gm of a gamma-alumina (LaRoche VGL) was loaded into each bottle. The alumina samples had been previously dried at 110° C overnight. An amount of 0.278 gms of deionized water was added dropwise to one reactor. An amount of 0.988 gms of methanol was added to another reactor. These amounts were calculated to be 10% of the catalyst plus water equivalent. The remaining reactor was used as a control reactor. Isobutane (246 cc) was added to each bottle; BF₃ was added (with stirring) until the pressure reached a constant 30 psig (207 kPa). A feedstock of 2/1: isobutane/2-butene was continuously added at a rate of 1.6 cc/minute. The reaction continued for about 75 minutes after which samples of the reactor liquids were extracted and analyzed using a gas-liquid chromatograph. The conversion of olefin was more than 99% in each case. Other reaction conditions and a summary of reaction results are shown in the following table:

| Reaction Conditions | Alumina | Alumina w/H₂O | Alumina w/CH₃OH |
|---|---|---|---|
| Reaction temperature (°C) | 0 | 0 | 0 |
| Pressure (psig) (kPa) | 30.0 (207) | 30.0 (207) | 30.0 (207) |
| WHSV | 5.653 | 5.526 | 5.526 |
| I/O (w/w) | 10.3 | 10.63 | 10.63 |

| Product | | | |
|---|---|---|---|
| C₅-C₇ | 2.29% | 2.80% | 14.85% |
| C₈ (saturated) | 94.61% | 89.81% | 64.67% |
| C₁₂⁺ | 2.52% | 6.81% | 13.58% |
| TMP/C₈ | 98.74% | 98.83% | 96.18% |
| Yield (w/w) | 2.19 | 2.14 | 1.87 |
| RON | 100.05 | 98.19 | 93.49 |
| R+M/2 | 98.32 | 96.06 | 92.74 |

It is clear that neither water nor methanol created any advantage in the operation of the process in producing a gasoline alkylate. The gross amounts of C₈ produced were smaller than for the inventive alumina; the amount of undesirable C₁₂⁺ were two to four times higher than for the inventive alumina. The yields were lower and, probably most importantly, the octane values of the comparative products were significantly lower.

### Example 4

This example shows the suitability of the inventive catalyst (gamma-alumina, LaRoche GL) for a variety of olefin feedstocks. The following reaction conditions were used for the test series:

| | |
|---|---|
| Temperature | 0° C |
| Total pressure | 30 psig (207 kPa) |
| WHSV | 4 |

A semi-batch reactor was utilized in each run.

The olefin feedstocks were mixtures which were chosen to allow us to identify desirable and undesirable combinations of feed materials. The mixtures were:

| Mixture No. | 1-C₄⁼ | i-C₄⁼ | C₃⁼ | 60/40 mixture of cis/trans 2-C₄⁼ |
|---|---|---|---|---|
| 1 | 25 | 25 | 20 | 30 |
| 2 | 10 | 25 | 20 | 45 |
| 3 | 25 | 10 | 20 | 45 |
| 4 | 10 | 10 | 20 | 60 |
| 5 | 25 | 25 | 05 | 45 |
| 6 | 10 | 25 | 05 | 60 |
| 7 | 25 | 10 | 05 | 60 |
| 8 | 10 | 10 | 05 | 75 |

The products made were analyzed using gas-liquid chromatography and their respective octane numbers calculated as follows:

| Mixture No. | C₅-C₇ | C₈ | C₁₂⁺ | TMP/C₈ | RON | R+M/2 |
|---|---|---|---|---|---|---|
| 1 | 14.4 | 55.0 | 23.7 | 94.1 | 86.3 | 86.3 |
| 2 | 15.3 | 52.9 | 25.9 | 93.6 | 86.3 | 87.1 |
| 3 | 15.4 | 58.6 | 20.2 | 94.9 | 88.7 | 87.9 |
| 4 | 14.5 | 60.8 | 17.0 | 95.1 | 90.9 | 90.1 |
| 5 | 10.2 | 66.4 | 17.9 | 92.6 | 89.9 | 88.9 |
| 6 | 7.3 | 62.9 | 25.3 | 95.1 | 89.8 | 89.0 |
| 7 | 6.4 | 74.9 | 16.1 | 94.7 | 92.1 | 90.5 |
| 8 | 6.8 | 71.9 | 11.6 | 96.3 | 93.1 | 91.9 |

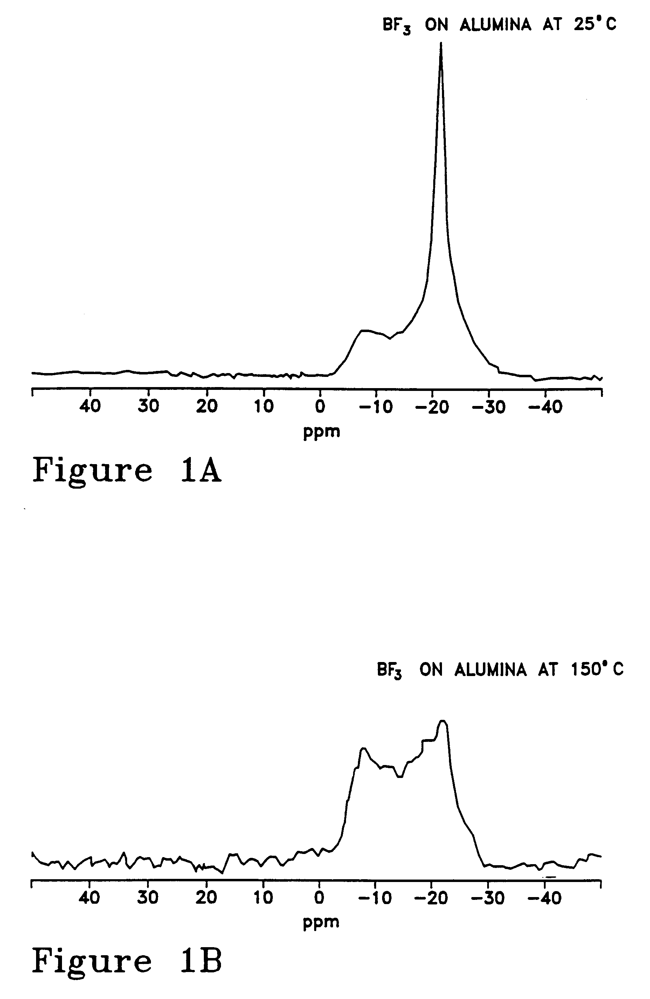

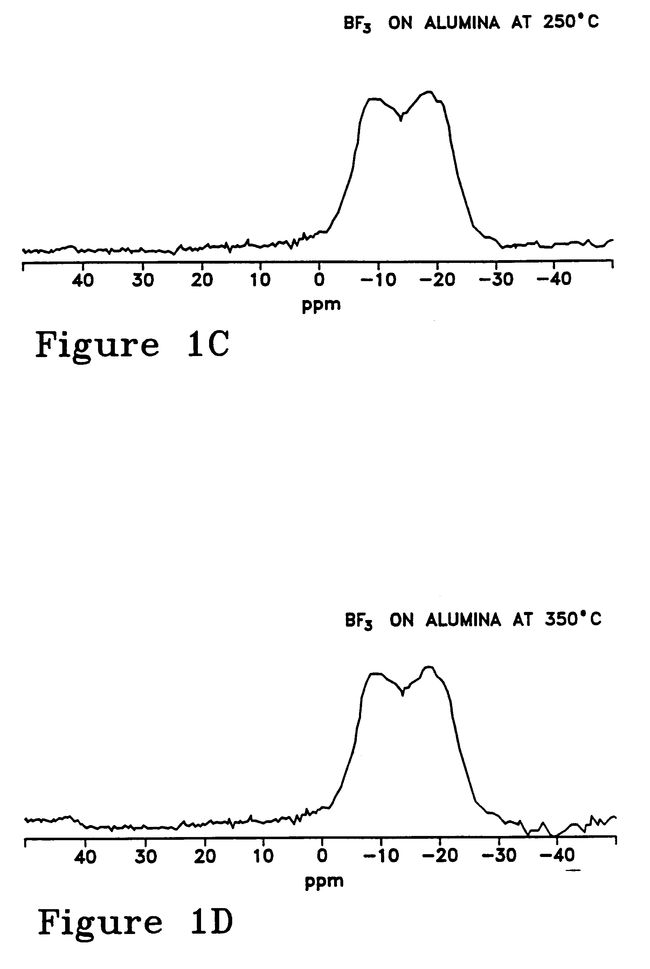

These data show that increases in isobutene and propylene feed concentrations directionally cause the inventive alkylation process to produce lower alkylate C₈ content. As shown in Figure 1, smaller amounts of either C₃⁼ or i-C₄⁼ cause no more harm to alkylate quality but are generally undesirable if extremely high octane alkylates are necessary.

### Example 5

This example demonstrates the performance of the transition alumina/BF₃ catalysts in reacting isobutane with butenes to form high octane product under conditions of high space velocity and low paraffin/olefin feed ratios.

A sample of gamma-alumina (VGL, LaRoche) was dried overnight at 110° C and loaded into the semi-continuous reactor unit described in Example 1. The catalyst was purged with dry inert gas and cooled to 0° C. Isobutane was added to the reactor and then the system was exposed to BF₃ under stirring conditions until an equilibrium pressure of 30 psig (207 kPa) was achieved. A feed comprising pure trans-2-butene was then pumped into the reactor under vigorous stirring conditions over a period of 60 minutes; samples were obtained periodically during the run. The results are summarized in the table below:

| | | |
|---|---|---|
| Catalyst charge (g) | 2.5 | |
| Temperature (°C) | 0 | |
| i-C₄ initial charge (ml) | 300 | |
| Olefin feed | Trans-2-butene | |
| Space velocity (WHSV) | 26.4 | |
| Run time (minutes) | 30 | 60 |
| Equivalent external i-C₄/C₄⁼ | 5.4 | 2.6 |
| Butene conversion (%) | 100 | 100 |

| Product analysis (weight %): | | |
|---|---|---|
| C₅-C₇ | 3.2 | 4.7 |
| C₈ saturates | 91.1 | 81.9 |
| C₉⁺ | 5.7 | 13.4 |
| TMP/C₈ total (%) | 97.6 | 96.6 |
| RON | 99.0 | 96.8 |
| Octane, R+M/2 | 97.0 | 95.3 |

### Example 6

This example shows the utility of the catalyst system on a feed obtained from a refinery MTBE unit. The feed, containing minor amounts of butadiene and isobutene, was introduced into a bed of a commercial hydroisomerization catalyst (0.3% Pd on Al₂O₃) at 400 cc/hr, 80° C, and 350 psig (2413 kPa) along with 14 sccm H₂. The molar ratio of H₂:butadiene was 6:1. The thusly treated feed, containing no butadiene and 0.52 % (molar) of isobutene, was mixed with an appropriate amount of isobutane. The mixture had the following approximate composition:

| Component | mole % |
|---|---|
| propylene | 0.02 |
| propane | 0.13 |
| isobutane | 80.14 |
| isobutene | 0.52 |
| 1-butene | 0.75 |
| butadiene | --- |
| n-butane | 3.84 |
| t-butene-2 | 8.23 |
| c-butene-2 | 4.05 |
| 3-methyl-1-butene | 0.01 |
| isopentane | 1.73 |
| 1-pentene | 0.01 |
| 2-methyl-1-butene | 0.03 |
| n-pentane | 0.08 |
| t-2-pentene | 0.18 |
| c-2-pentene | 0.06 |
| 2-methyl-2-butene | 0.23 |

This mixture had an isoparaffin/olefin ratio of 6.10 and an isobutane/olefin ratio of 5.69.

The mixture was then admitted to a pair of continuous laboratory reactors each containing 280 cc of liquid and containing 5.04 g of catalyst. The temperature was maintained at 0° F. The WHSV for the reactor was 4.3 hr⁻¹ and the LHSV was 1.07 hr⁻¹. The catalyst was a gamma alumina (LaRoche VGL) and was prepared by adding the proper amount to the reactors along with a small amount of isobutane, pressuring the reactor to about 40 psig (276 kPa) of BF₃, and maintaining that pressure for the duration of the test.

The test was run for 41 hours total time. The catalyst was regenerated four times during the run by rinsing the catalyst in 200cc of trimethyl pentane, heating to 150° C in air for 45 minutes to volatilize a portion of the reaction product on the catalyst, and heating the catalyst to 600° C in air for 60 minutes to oxidize the remaining hydrocarbonaceous materials. Small amounts of the catalyst were added as necessary with the regenerated catalyst to restore the catalyst to its proper amount upon return to the reactor (0.41 g @ cycle 2, 0.97 g @ cycle 3, 0.0 g @ cycle 4, and 0.47 g @ cycle 5). About 4.5 liters (3.2 kg) of stripped C₅₊ alkylate was collected having about 7.6% C₅₋₇, 81.2% C₈, 4.4% C₉₋₁₁, and 6.8% C₁₂ (all by weight). Using the Hutson method discussed above, the octanes were calculated to be: RON = 96.6, MON = 93.3, and the (R+M)/2 = 94.95. The product was then engine-tested using API methodology and the octanes were measured to be: RON = 98.7, MON = 93.85. The resulting (R + M)/2 = 96.28. The Hutson method clearly underestimated the RON octane values for this process.

### Example 7

This example shows the preparation of a number of BF₃/alumina-based catalyst components. One is made in accord with this invention and three are comparative samples. Each of the samples was then tested in an alkylation reaction using model feeds and isobutane and butenes.

All four gamma-alumina samples (LaRoche-Versal GL) were infused with BF₃ in a Cahn balance. Use of a Cahn balance allowed close control of the temperature at which the alumina contacted the BF₃ and further allowed the weight gain to be measured during the treatment The four samples were treated with BF₃ respectively at 25° C, 150° C, 250° C, and 350° C. The amount of BF₃ in the samples was 17.4%, 17.3%, 13.7% and 13.6% (by weight). A sample of each of the catalyst components was removed and analyzed using ¹¹B-MAS-NMR. The results of these analyses are shown in the figures: Figure 1A shows the treated alumina; Figures 1B, 1C, and 1D show the respective data from the 150° C, 250° C, and 350° C treated aluminas. In Figure 1A, there is a pronounced sharp peak at about -21.27 ppm (relative to boric acid) suggesting significant tetragonal boron content. The other three NMR plots do not show such a sharp peak. Instead, the data suggest the presence of substantial trigonal boron.

The four gamma-alumina samples (LaRoche-Versal GL) were then charged to a semi-batch reactor having an internal volume of about 500 cc. The reactor temperature was controllable over the range of -5° C to 40° C. For initial catalyst treatment, the reactor containing the catalyst was purged with an inert gas and cooled to about 0° C. About 275 cc of isobutane were added to the reactor. After a brief degassing, BF₃ was added batchwise. Additional infusions of BF₃ are made until the pressure in the reactor no longer drops. The BF₃ saturation equilibrium pressure was about 40 psig (276 kPa). The liquid phase concentration of BF₃ was about 1.5%.

A mixture of isobutane/trans-2-butene was fed to the reactor. At the completion of the reaction, alkylate was removed and analyzed by gas-liquid chromatography. The RON's were calculated from the gas-liquid chromatography data using the well-known correlations in Hutson and Logan, "Estimate Alkyl Yield and Quality", Hydrocarbon Processing, September, 1975, pp. 107-108. The summary of the experiments and results is shown in the table below:

| Feed and Operating Conditions | | | | |
|---|---|---|---|---|
| Sample No. | 1 | 2 | 3 | 4 |
| Al₂O₃/BF₃ treatment temp. (° C) | 25° | 150° | 250° | 350° |
| catalyst charge (gm) | 1.21 | 1.21 | 1.21 | 1.21 |
| reaction temp. (° C) | 0 | 0 | 0 | 0 |
| reaction pressure (psig) (kPa) | 30 (207) | 30 (207) | 30 (207) | 30 (207) |
| iC₄/olefin (w/w) | 5.98 | 5.93 | 5.93 | 5.93 |
| i-C₄ (cc) | 68.6 | 68.6 | 68.6 | 68.6 |
| feed (cc) | 306.4 | 306.4 | 306.4 | 306.4 |
| WHSV | 14.869 | 15.559 | 15.718 | 17.307 |

| Product Summary | | | | |
|---|---|---|---|---|
| Sample No. | 1 | 2 | 3 | 4 |
| Total alkylate | 27.63% | 19.97% | 11.48% | 5.62% |
| Butene Conversion | 100.0% | 86.15% | 69.09% | 67.03% |
| C₅₋₇ hydrocarbon | 1.51% | 2.53% | 2.55% | 0.83% |
| C₈ hydrocarbon | 93.33% | 87.58% | 85.14% | 70.56% |
| C₉₋₁₁ hydrocarbon | 0.49% | 3.12% | 6.45% | 13.90% |
| C₁₂₊ hydrocarbon | 4.67% | 6.77% | 5.86% | 14.72% |
| TMP/C₈ | 97.54% | 98.27% | 96.70% | 97.55% |
| Yield (w/w) | 1.94 | 1.40 | 0.80 | 0.39 |
| RON | 99.26 | 98.09 | 97.57 | 92.23 |
| MON | 94.88 | 93.73 | 93.16 | 90.07 |
| (R+M)/2 | 97.07 | 95.91 | 95.36 | 91.15 |
| 2,2,4/2,3,4 | 0.81 | 0.54 | 0.48 | 0.33 |

It is clear from the data that the catalyst component treated with BF₃ at the lower temperature is superior in operation in most practical aspects (conversion, C₈ production, alkylate yield, RON, MON, etc.) than the other materials.

## Claims

1. An alkylation process comprising the steps of:
(A) contacting a mixture comprising isoparaffins and olefins with an alkylation catalyst system which comprises:
(i) an alumina alkylation catalyst component comprising a transition alumina which has been contacted under substantially anhydrous conditions with a Lewis acid at a treatment temperature below 150°C to produce an alkylation catalyst containing Lewis acid;
and
(ii) an amount of that free Lewis acid sufficient to maintain the Lewis acid concentration of the alumina alkylation catalyst component, wherein the total amount of Lewis acid in the alumina surface is between 0.5% and 40% by weight of the catalyst;
to produce an alkylate stream, and
(B) separating the alkylate stream from the alumina based alkylation catalyst.

2. A process according to claim 1, where alkylation conditions include a temperature in the range of -30°C to 50°C.

3. A process according to claim 1 or 2, where the isoparaffin and olefin mixture comprise 2-butene and isoparaffin.

4. A process according to any of claims 1 to 3, where the contacting step is carried out in the substantial absence of isobutylene.

5. A process according to any of claims 1 to 4, where the isoparaffin comprises isobutane.

6. A process according to any of claims 1 to 5, where alkylation conditions include a WHSV of between 0.5 and 30.0.

7. A process according to any of claims 1 to 6, where the ratio of C₄-C₁₀ isoparaffins to C₃-C₅ olefins is in the range of from 1 to 50.

8. A process according to any of claims 1 to 7, additionally including the step of mixing the alkylate stream with other hydrocarbons to produce a gasoline blending component or gasoline.

9. An alkylation catalyst system suitable for use in the alkylation process of claim 1, said catalyst system comprising:
(i) an alumina alkylation catalyst component comprising a transition alumina which has been contacted under substantially anhydrous conditions with a Lewis acid at a treatment temperature of less than 30°C to produce an alkylation catalyst containing Lewis acid;
and
(ii) an amount of free Lewis acid sufficient to maintain the Lewis acid concentration of the alumina alkylation catalyst component, wherein the total amount of Lewis acid in the alumina surface is between 0.5% and 40% by weight of the catalyst.

10. A catalyst system according to claim 9, where the transition alumina is gamma-alumina, eta-alumina, theta-alumina, chi-alumina, rho-alumina, or a mixture thereof.

11. A catalyst system according to claim 10, where the transition alumina is gamma-alumina or eta-alumina or a mixture thereof.

12. A catalyst system according to any one of claims 9 to 11, where the Lewis acid contains boron.

13. A catalyst system according to any one of claims 9 to 12, where the Lewis acid is BF₃, BCl₃, BBr₃ or BI₃.

14. A catalyst system according to claim 13, where the Lewis acid is BF₃.

15. A catalyst system according to any one of claims 9 to 11, where the Lewis acid is SbF₅, AlCl₃, AlBr₃, TiBr₄, TiCl₄, TiCl₃, ZrCl₄, PF₅, FeCl₃ or FeBr₃.

16. A catalyst system according to claim 15, where the Lewis acid is SbF₅ or AlCl₃.

17. A catalyst system according to any one of claims 9 to 16, containing substantially no metals or semi-metals in catalytic amounts other than aluminium and the semi-metal boron.

18. A catalyst system according to any one of claims 9 to 17, additionally comprising isobutane and butylene.

19. A process according to any one of claims 1 to 8, in which the alkylation catalyst system is as claimed in any one of claims 9 to 18.

## Patentansprüche

1. Alkylierungsverfahren, unfassend die Schritte:
(A) Inkontaktbringen eines Gemischs, umfassend Isoparaffine und Olefine, mit einem Alkylierungskatalysatorsystem, welches umfaßt:
(i) eine Aluminiumoxid-Alkylierungskatalysatorkomponente, umfassend ein Übergangs-Aluminiumoxid, unter im wesentlichen wasserfreien Bedingungen bei einer Behandlungstemperatur unter 150°C mit einer Lewissäure in Kontakt gebracht, um einen Lewissäure enthaltenden Alkylierungskatalysator zu erzeugen;
und
(ii) eine Menge dieser freien Lewissäure, die ausreicht, um die Lewissäurekonzentration der Aluminiumoxid-Alkylierungskatalysatorkomponente aufrechtzuerhalten, wobei die Gesamtmenge an Lewissäure in der Aluminiumoxid-Oberfläche zwischen 0,5 Gew.-% und 40 Gew.-% des Katalysators liegt;
um einen Alkylatstrom zu erzeugen, und
(B) Trennen des Alkylatstroms von dem Alkylierungskatalysator auf Aluminiumoxid-Basis.

2. Verfahren nach Anspruch 1, wobei die Alkylierungsbedingungen eine Temperatur im Bereich von -30°C bis 50°C beinhalten.

3. Verfahren nach Anspruch 1 oder 2, wobei das Isoparaffin- und Olefingemisch 2-Buten und Isoparaffin umfaßt.

4. Verfahren nach einem der Ansprüche 1 bis 3, wobei der Kontaktschritt im wesentlichen in Abwesenheit von Isobutylen durchgeführt wird.

5. Verfahren nach einem der Ansprüche 1 bis 4, wobei das Isoparaffin Isobutan umfaßt.

6. Verfahren nach einem der Ansprüche 1 bis 5, wobei die Alkylierungsbedingungen eine WHSV zwischen 0,5 und 30,0 einschließen.

7. Verfahren nach einem der Ansprüche 1 bis 6, wobei das Verhältnis von C₄-C₁₀-Isoparaffinen zu C₃-C₅-Olefinen im Bereich von 1 bis 50 liegt.

8. Verfahren nach einem der Ansprüche 1 bis 7, das zusätzlich den Schritt des Mischens des Alkylatstroms mit anderen Kohlenwasserstoffen beinhaltet, um eine Benzinmischkomponente oder Benzin herzustellen.

9. Alkylierungskatalysatorsystem, das zur Verwendung in dem Alkylierungsverfahren nach Anspruch 1 geeignet ist, wobei das Katalysatorsystem umfaßt:
(i) eine Aluminiumoxid-Alkylierungskatalysatorkomponente, umfassend ein Übergangs-Aluminiumoxid, unter im wesentlichen wasserfreien Bedingungen bei einer Behandlungstemperatur von unter 30°C mit einer Lewissäure in Kontakt gebracht um einen Lewissäure enthaltenden Alkylierungskatalysator zu erzeugen;
und
(ii) eine Menge an freier Lewissäure, die ausreicht, um die Lewissäurekonzentration der Aluminiumoxid-Alkylierungskatalysatorkomponente aufrechtzuerhalten, wobei die Gesamtmenge an Lewissäure in der Aluminiumoxid-Oberfläche zwischen 0,5 Gew.-% und 40 Gew.-% des Katalysators liegt.

10. Katalysatorsystem nach Anspruch 9, wobei das Übergang-Aluminiumoxid Gamma-Aluminiumoxid, Eta-Aluminiumoxid, Theta-Aluminiumoxid, Chi-Aluminiumoxid, Rho-Aluminiumoxid oder ein Gemisch davon ist.

11. Katalysatorsystem nach Anspruch 10, wobei das Übergangs-Aluminiumoxid Gamma-Aluminiumoxid oder Eta-Aluminiumoxid oder ein Gemisch davon ist.

12. Katalysatorsystem nach einem der Ansprüche 9 bis 11, wobei die Lewissäure Bor enthält.

13. Katalysatorsystem nach einem der Ansprüche 9 bis 12, wobei die Lewissäure BF₃, BCl₃, BBr₃ oder BI₃ ist.

14. Katalysatorsystem nach Anspruch 13, wobei die Lewissäure BF₃ ist.

15. Katalysatorsystem nach einem der Ansprüche 9 bis 11, wobei die Lewissäure SbF₅, AlCl₃, AlBr₃, TiBr₄, TiCl₄, TiCl₃, ZrCl₄, PF₅, FeCl₃ oder FeBr₃ ist.

16. Katalysatorsystem nach Anspruch 15, wobei die Lewissäure SbF₅ oder AlCl₃ ist.

17. Katalysatorsystem nach einem der Ansprüche 9 bis 16, das im wesentlichen keine Metalle oder Halbmetalle in katalytischen Mengen enthält, die verschieden von Aluminium und dem Halbmetall Bor sind.

18. Katalysatorsystem nach einem der Ansprüche 9 bis 17, das zusätzlich Isobutan und Butylen umfaßt.

19. Verfahren nach einem der Ansprüche 1 bis 8, wobei das Alkylierungskatalysatorsystem gemäß einem der Ansprüche 9 bis 18 ist.

## Revendications

1. Procédé d'alkylation comprenant les étapes consistant à:
(A) mettre en contact un mélange comprenant des isoparaffines et des oléfines avec un système de catalyseur d'alkylation qui comprend:
(i) un composant de catalyseur d'alkylation à base d'alumine comprenant une alumine de transition qui a été mise en contact, dans des conditions pratiquement anhydres, avec un acide de Lewis à une température de traitement inférieure à 150°C pour produire un catalyseur d'alkylation contenant de l'acide de Lewis;
et;
(ii) une quantité de cet acide de Lewis libre suffisante pour maintenir la concentration en acide de Lewis du composant de catalyseur d'alkylation à base d'alumine, où la quantité totale d'acide de Lewis dans la surface de l'alumine représente entre 0,5% et 40% en poids du catalyseur;
pour produire un courant d'alkylates, et
(B) séparer le courant d'alkylates du catalyseur d'alkylation à base d'alumine.

2. Procédé selon la revendication 1, dans lequel les conditions d'alkylation englobent une température située dans l'intervalle allant de -30°C à 50°C.

3. Procédé selon la revendication 1 ou 2, dans lequel le mélange d'isoparaffines et d'oléfines comprend du 2-butène et de l'isoparaffine.

4. Procédé selon l'une quelconque des revendications 1 à 3, dans lequel on effectue l'étape de mise en contact en l'absence pratiquement totale d'isobutylène.

5. Procédé selon l'une quelconque des revendications 1 à 4, dans lequel l'isoparaffine comprend de l'isobutane.

6. Procédé selon l'une quelconque des revendications 1 à 5, dans lequel les conditions d'alkylation englobent une WHSV (vitesse spatiale horaire en poids) valant entre 0,5 et 30,0.

7. Procédé selon l'une quelconque des revendications 1 à 6, dans lequel le rapport des isoparaffines en C₄-C₁₀ aux oléfines en C₃-C₅ est situé dans l'intervalle allant de 1 à 50.

8. Procédé selon l'une quelconque des revendications 1 à 7, comprenant en outre l'étape consistant à mélanger le courant d'alkylates avec d'autres hydrocarbures pour produire un composant de mélange d'essence ou de l'essence.

9. Système de catalyseur d'alkylation convenant pour être utilisé dans le procédé d'alkylation selon la revendication 1, ledit système de catalyseur comprenant:
(i) un composant de catalyseur d'alkylation à base d'alumine comprenant une alumine de transition qui a été mise en contact, dans des conditions pratiquement anhydres, avec un acide de Lewis à une température de traitement inférieure à 30°C pour produire un catalyseur d'alkylation contenant de l'acide de Lewis;
et;
(ii) une quantité d'acide de Lewis libre suffisante pour maintenir la concentration en acide de Lewis du composant de catalyseur d'alkylation à base d'alumine, où la quantité totale d'acide de Lewis dans la surface de l'alumine représente entre 0,5% et 40% en poids du catalyseur.

10. Système de catalyseur selon la revendication 9, dans lequel l'alumine de transition est de l'alumine gamma, de l'alumine êta, de l'alumine thêta, de l'alumine chi, de l'alumine rho ou un mélange de celles-ci.

11. Système de catalyseur selon la revendication 10, dans lequel l'alumine de transition est de l'alumine gamma ou de l'alumine êta ou un mélange de celles-ci.

12. Système de catalyseur selon l'une quelconque des revendications 9 à 11, dans lequel l'acide de Lewis contient du bore.

13. Système de catalyseur selon l'une quelconque des revendications 9 à 12, dans lequel l'acide de Lewis est BF₃, BCl₃, BBr₃ ou BI₃.

14. Système de catalyseur selon la revendication 13, dans lequel l'acide de Lewis est BF₃.

15. Système de catalyseur selon l'une quelconque des revendications 9 à 11, dans lequel l'acide de Lewis est SbF₅, AlCl₃, AlBr₃, TiBr₄, TiCl₄, TiCl₃, ZrCl₄, PF₅, FeCl₃ ou FeBr₃.

16. Système de catalyseur selon la revendication 15, dans lequel l'acide de Lewis est SbF₅ ou AlCl₃.

17. Système de catalyseur selon l'une quelconque des revendications 9 à 16, ne contenant pratiquement pas de métaux ou d'éléments semi-métalliques en quantités catalytiques autres que l'aluminium et l'élément semi-métallique bore.

18. Système de catalyseur selon l'une quelconque des revendications 9 à 17, comprenant en outre de l'isobutane et du butylène.

19. Procédé selon l'une quelconque des revendications 1 à 8, dans lequel le système de catalyseur d'alkylation est tel que revendiqué dans l'une quelconque des revendications 9 à 18.
